# EUROPEAN PATENT SPECIFICATION

(11) **EP 1 993 154 B1**
(45) Date of publication and mention of the grant of the patent: **25.08.2010**
(21) Application number: 08008984.0
(22) Date of filing: 15.05.2008
(51) Int. Cl.: H01L 51/50, H01L 51/54

(54) **Electron transporting materials and organic light-emitting devices therewith**
Elektronentransportmaterialien und organische lichtemittierende Vorrichtungen damit
Matériaux de transport d'électrons et dispositifs associés électroluminescents organiques

(30) Priority: 16.05.2007 JP 2007130419; 31.03.2008 JP 2008090810
(43) Date of publication of application: 19.11.2008
(73) Proprietor: Yamagata Promotional Organization for Industrial Technology, Yamagata-shi, Yamagata 990-2473 (JP)
(72) Inventor: Kimura, Masato, Yamagata-shi Yamagata 9902473 (JP); Oda, Atsushi, Yamagata-shi Yamagata 9902473 (JP); Kido, Junji, Yamagata-shi Yamagata 9902473 (JP)
(74) Representative: HOFFMANN EITLE

(56) References cited:
- JP-A- 2001 267 080
- S. NAKA, ET. AL.: "High electron mobility in bathophenanthroline" APPLIED PHYSICS LETTERS, vol. 76, no. 2, 2000, pages 197-199, XP002495969 AIP
- C. ADACHI, ET. AL.: "High-efficiency organic electrophosphorescent devices with tris(2-phenylpyridine)iridium doped into electron-transporting materials" APPLIED PHYSICS LETTERS, vol. 77, no. 6, 2000, pages 904-906, XP002495970 AIP
- J. KIDO, ET. AL.: "Bright organic electroluminescent devices having a metal-doped electron-injecting layer" APPLIED PHYSICS LETTERS, vol. 73, no. 20, 1998, pages 2866-2868, XP002495971 AIP

## Description

### BACKGROUND OF THE INVENTION

### Field of the Invention

The present invention relates to novel electron transporting materials including phenanthroline derivatives and to organic light-emitting devices therewith.

### Description of the Related Art

Organic light-emitting devices, which are self-luminescence devices using organic compounds as luminescent materials, are characterized in that they are suitable for movie display because of their ability to emit light at high speed and that they can form thin display panels because of their simple structure. Organic light-emitting devices having such good characteristics are becoming widespread in daily-life applications such as cellular phones and car displays.

Organic light-emitting devices have a basic layered structure of anode/light-emitting layer/cathode, in which a layer containing a hole or electron transporting material having the function of transporting holes or electrons is generally placed in order to increase the efficiency and the life.

Conventionally, tris(8-quinolinolato)aluminum represented by Chemical Formula 1 below (hereinafter abbreviated as Alq₃) is widely used as the electron transporting material. However, it cannot emit blue light, although it emits green light.

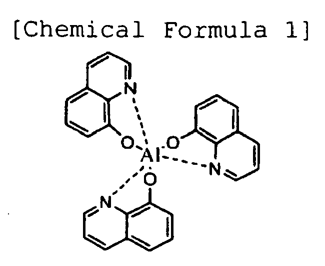

Therefore, new types of compounds such as bathophenanthroline represented by Chemical Formula 2 below and bathocuproin represented by Chemical Formula 3 below are proposed as electron transporting materials having a large energy gap.

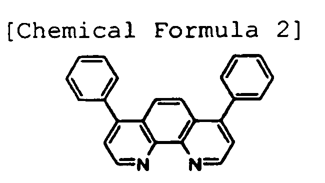

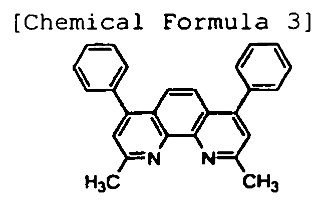

For example, bathophenanthroline is known to have high electron mobility and characterized in that it can achieve a reduction in voltage and an increase in efficiency when doped with cesium (see Japanese Patent No. 3562652; Japanese Patent Application Laid-Open (JP-A) No. 2001-267080; Shigeki Naka et al., Applied Physics Letters, 76(2), P. 197; Junji Kido et al., Applied Physics Letters, 73(20), p. 2866; and Kishigami at al., Kobunshi Toronkai, 2000, Vol. 49, No. 11, p. 3385).

Bathocuproin is also used as a hole blocking material for phosphorescent devices (see for example Chihaya Adachi et al., Applied Physics Letters, 77. p. 904).

However, the conventional materials described in each patent or non-patent document have problems such as a planar structure, high crystallinity and difficulty in retaining an amorphous thin film. The conventional materials also have low heat resistance so that degradation of devices therewith can be enhanced by Joule heat generated in the devices or by the temperature of the external environment.

Such low heat resistance of components of organic light-emitting devices is a significant problem with facsimiles, copying machines, backlights for liquid crystal displays, and light sources such as lights and also undesirable for display devices such as full-color flat-panel displays.

Therefore, there has been a demand for the development of electron transporting materials having thermal characteristics higher than those of conventional ones as described above.

### SUMMARY OF THE INVENTION

The present invention has been made in order to solve the technical problems described above, and an object of the invention is to provide novel electron transporting materials having high thermal characteristics and to provide organic light-emitting devices using such materials.

The invention is directed to an electron transporting material that is a compound represented by Chemical Formula 4:

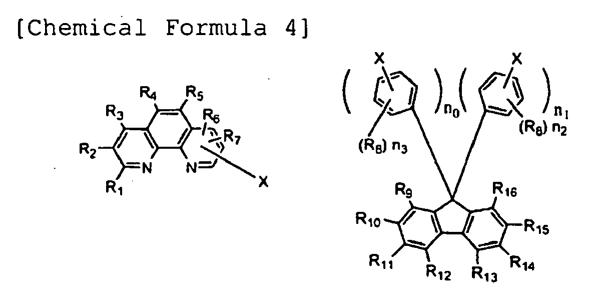

In Chemical Formula 4, R₁ to R₁₆ are each selected from hydrogen, alkyl, cycloalkyl, alkenyl, cycloalkenyl, alkynyl, aralkyl, aralkenyl, aralkynyl, aryl, cycloaryl, halogen, haloalkyl, haloalkenyl, haloalkynyl, haloaryl, heterocycle, alkoxy, aryloxy, hydroxyl, amino, alkylamino, arylamino, cyano, nitro, alkylthio, arylthio, aldehyde, carbonyl, carboxyl, ester, carbamoyl, silyl, and siloxanyl groups and may be the same or different; the position of the substituent R₆ or R₇ depends on the position of the linkage between the phenanthrolinyl group and the fluorenyl group; X represents a single bond between the phenanthrolinyl group and the fluorenyl group; n₀ and n₁ each independently represent an integer of 0 to 2; and n₂ and n₃ each represent the number of the substituents and each independently represent an integer of 1 to 4.

Electron transporting materials including such a compound can form organic light-emitting devices with high thermal characteristics.

Among the compounds represented by the above formula, diphenanthrolinylfluorene (hereinafter abbreviated as DPF) represented by Chemical Formula 5 below or m-DPF represented by Chemical Formula 6 below is particularly preferred.

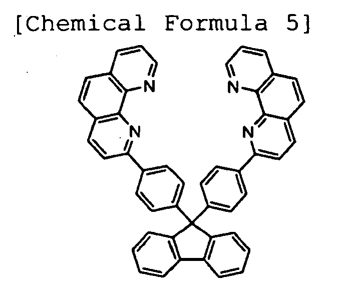

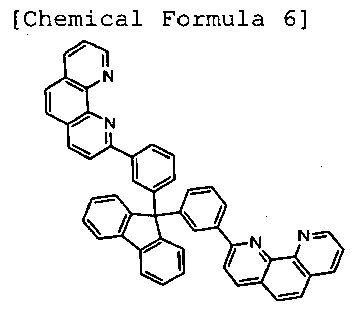

The invention is also directed to an organic light-emitting device including a layer containing the electron transporting material defined above.

High heat-resistant devices can be formed using the electron transporting material of the invention.

The invention is also directed to an organic light-emitting device including a first electrode, a light-emitting layer and a second electrode placed in this order, wherein the light-emitting layer includes a hole-transporting luminescent material and the electron transporting material defined above.

In another embodiment of the invention, there is provided an organic light-emitting device including a first electrode, a light-emitting layer, a hole blocking layer, and a second electrode placed in this order, wherein the hole blocking layer includes the electron transporting material defined above.

In another embodiment of the invention, there is provided an organic light-emitting device including a first electrode, a hole transporting layer, a light-emitting layer, a hole blocking layer, and a second electrode placed in this order, wherein the hole blocking layer includes the electron transporting material defined above.

The organic light-emitting device may further include an electron transporting layer that is placed between the hole blocking layer and the second electrode and adjacent to the hole blocking layer or may further include a hole injecting layer that is placed between the first electrode and the hole transporting layer and adjacent to the hole transporting layer.

The hole blocking layer may be doped with a metal or a metal complex. The light-emitting layer may include a molecular dispersion of components including at least a hole-transporting luminescent material and the electron transporting material defined above or may include a luminescent material as a dopant and a molecular dispersion of components including at least a hole-transporting luminescent material and the electron transporting material defined above.

The first electrode preferably includes a transparent electrically-conductive thin film formed on a transparent substrate.

As described above, the electron transporting material of the invention can function in an electron transporting layer or a hole blocking layer and have high heat resistance and form stable thin films. The electron transporting material of the invention can also provide a significant reduction in voltage and a significant increase in device efficiency as compared with a typical conventional electron transporting material, Alq₃ and therefore can increase the reliability of organic light-emitting devices.

Therefore, the organic light-emitting device of the invention is expected to be applicable to applications requiring long-term, stable lighting, such as flat panel displays for OA computers and wall-hung TVs, lighting fixtures, light sources for copying machines, light sources for which surface emitting characteristics are exploited, such as backlights for liquid crystal displays and measuring instruments, display panels, and marker lamps.

### BRIEF DESCRIPTION OF THE DRAWINGS

Fig. 1 is a cross-sectional view schematically showing the layered structure of an organic light-emitting device according to Example 1 or 2;
Fig. 2 is a micrograph of the surface of a vapor-deposited DPB film immediately after vapor deposition;
Fig. 3 is a micrograph of the surface of a vapor-deposited DPF film three weeks after vapor deposition; and
Fig. 4 is a micrograph of the surface of a vapor-deposited m-DPF film three weeks after vapor deposition.

### DESCRIPTION OF THE PREFERRED EMBODIMENTS

The present invention is described more in detail below.

The electron transporting material of the invention is a compound represented by a general formula in Chemical Formula 4 shown above.

Such a phenanthroline derivative is a compound having a three-dimensional structure and high heat resistance. When used as an electron transporting material, it can improve the thermal characteristics of organic light-emitting devices more effectively than conventional materials.

In Chemical Formula 4, R₁ to R₁₆ are each selected from hydrogen, alkyl, cycloalkyl, alkenyl, cycloalkenyl, alkynyl, aralkyl, aralkenyl, aralkynyl, aryl, cycloaryl, halogen, haloalkyl, haloalkenyl, haloalkynyl, haloaryl, heterocycle, alkoxy, aryloxy, hydroxyl, amino, alkylamino, arylamino, cyano, nitro, alkylthio, arylthio, aldehyde, carbonyl, carboxyl, ester, carbamoyl, silyl, and siloxanyl and may be the same or different; the position of the substituent R₆ or R₇ depends on the position of the linkage between the phenanthrolinyl group and the fluorenyl group; X represents a single bond between the phenanthrolinyl group and the fluorenyl group; n₀ and n₁ each independently represent an integer of 0 to 2; and n₂ and n₃ each represent the number of the substituents and each independently represent an integer of 1 to 4.

Among the substituents, the alkyl group refers to a saturated aliphatic hydrocarbon group such as methyl, ethyl, propyl, and butyl and may be a straight or branched chain.

The cycloalkyl group refers to a saturated alicyclic hydrocarbon group such as cyclohexyl, norbornyl and adamantyl and may be substituted or unsubstituted.

The alkenyl group refers to an unsaturated, double bond-containing, aliphatic hydrocarbon group such as vinyl, allyl and butadienyl groups and may be a straight or branched chain.

The cycloalkenyl group refers to an unsaturated, double bond-containing, alicyclic hydrocarbon group such as cyclohexenyl group and cyclopentadienyl group and may be substituted or unsubstituted.

The alkynyl group refers to an unsaturated, triple bond-containing, aliphatic hydrocarbon group such as acetylenyl group.

The aralkyl group refers to an aromatic hydrocarbon group linked to a saturated aliphatic hydrocarbon moiety, such as benzyl and phenylethyl. The saturated aliphatic hydrocarbon group and the aromatic hydrocarbon group may be each substituted or unsubstituted.

The aralkenyl group refers to an aromatic hydrocarbon group linked to an unsaturated aliphatic hydrocarbon group, such as styryl and diphenylvinyl group. The unsaturated hydrocarbon group and the aromatic hydrocarbon group may be each substituted or unsubstituted.

The aralkynyl group refers to an aromatic hydrocarbon group linked to an unsaturated aliphatic hydrocarbon group, such as phenylacetylenyl, in which the aromatic hydrocarbon group may be substituted or unsubstituted.

The aryl group refers to an aromatic hydrocarbon group such as phenyl, naphthyl and anthranil, in which the aromatic hydrogen group may be substituted or unsubstituted.

The cycloaryl group refers to a saturated or unsaturated alicyclic hydrocarbon group-containing aromatic hydrocarbon group such as cyclophenyl and a tetrahydronaphthalene group, which may be substituted or unsubstituted.

The halogen group refers to any one of fluorine, chlorine, bromine, and iodine.

The haloalkyl, haloalkenyl or haloalkynyl group refers to an alkyl, alkenyl or alkynyl group partially or completely substituted with halogen, such as trifluoromethyl.

The haloaryl group refers to a halogen-substituted aromatic hydrocarbon group such as chlorophenyl, dichlorophenyl and chloronaphthyl. The remaining potentially-substituted moieties other than the halogen-substituted group may be substituted or unsubstituted.

The heterocycle group refers to a group containing carbon and any of nitrogen, oxygen and sulfur as ring members. Examples include triazine, oxazole, oxadiazole, thiazole, thiadiazole, furan, furazan, thiophene, pyran, pyrrole, pyrazole, imidazole, imidazolidine, imidazoline, chroman, coumarone, indole, indoline, isocoumarone, cinnoline, quinazoline, quinoxaline, phthalazine, phenothiazine, acridine, phenanthridine, quinoline, isoquinoline, naphthyridine, pyridine, pyrimidine, triazole, and carbazole. The heterocycle group may be substituted or unsubstituted.

The alkoxy group refers to a saturated aliphatic hydrocarbon group bonded through an ether linkage, such as methoxy group, and may be a straight or branched chain.

The aryloxy group refers to an aromatic hydrocarbon group bonded through an ether linkage, such as phenoxy, in which the aromatic hydrocarbon group may be substituted or unsubstituted.

The alkylamino group refers to an aliphatic hydrocarbon group linked to a nitrogen atom, such as dimethylamino and diethylamino, in which the aliphatic hydrocarbon group may be a straight or branched chain.

The arylamino group refers to an aromatic hydrocarbon group linked to a nitrogen atom, such as diphenylamino and ditolylamino, in which the aromatic hydrocarbon group may be substituted or unsubstituted.

The alkylthio group corresponds to an analog of the alkoxy group in which a sulfur atom is substituted for an oxygen atom.

The arylthio group corresponds to an analog of the aryloxy group in which a sulfur atom is substituted for an oxygen atom.

The silyl group refers to a silicon compound group such as trimethylsilyl.

The siloxanyl group refers to a silicon compound group bonded through an ether linkage, such as trimethylsiloxanyl.

Some examples of the structure of the compound represented by the general formula are illustrated below.

First, some examples of substituent-free basic skeletons with variable linkage positions are listed below under the title of Chemical Formula 7.

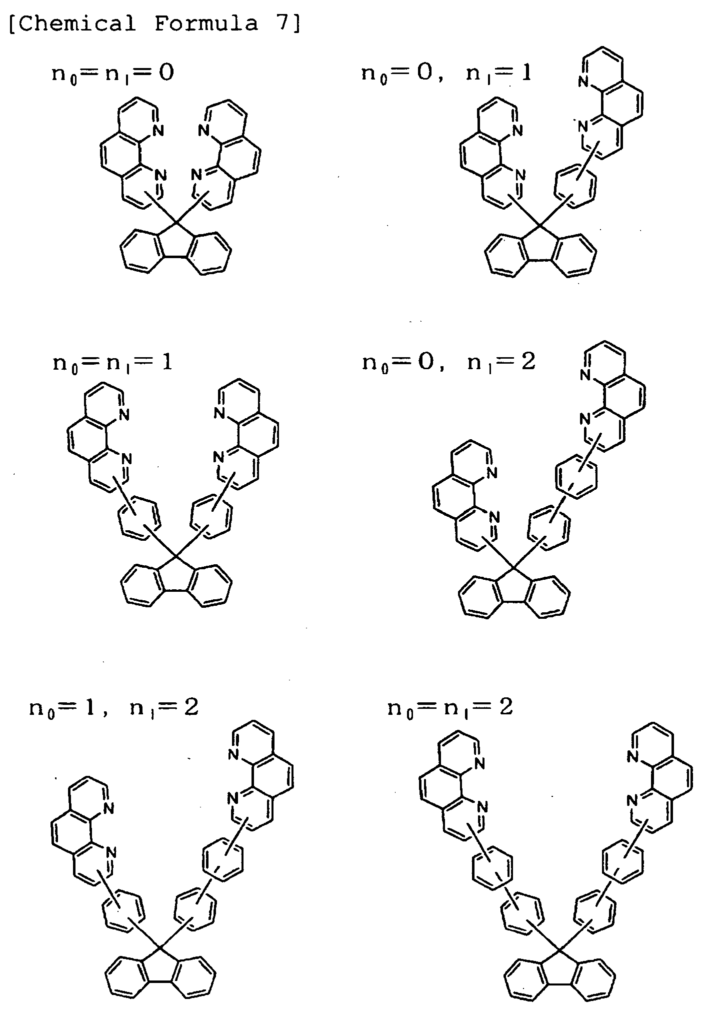

Concerning the basic skeletons shown under the title of Chemical Formula 7, some examples of basic skeletons with fixed linkage positions are further illustrated below under the titles of Chemical Formulae 8 to 12.

The linkage between the phenanthrolinyl group and the fluorenyl group in a position of primary choice may be a linkage between position 2 of the phenanthrolinyl group and the linking group substituted in para position between the phenanthrolinyl group and the fluorenyl group. The linkage between the phenanthrolinyl group and the fluorenyl group in a position of secondary choice may be a linkage between position 2 of the phenanthrolinyl group and the linking group substituted in ortho or meta position between the phenanthrolinyl group and the fluorenyl group. The linkage between the phenanthrolinyl group and the fluorenyl group in a position of tertiary or minor choice may be a linkage between position 3 or 4 of the phenanthrolinyl group and the linking group substituted in para, meta or ortho position between the phenanthrolinyl group and the fluorenyl group.

Basic skeletons having linkage positions of primary and secondary choices are illustrated below under the titles of Chemical Formulae 8 to 12.

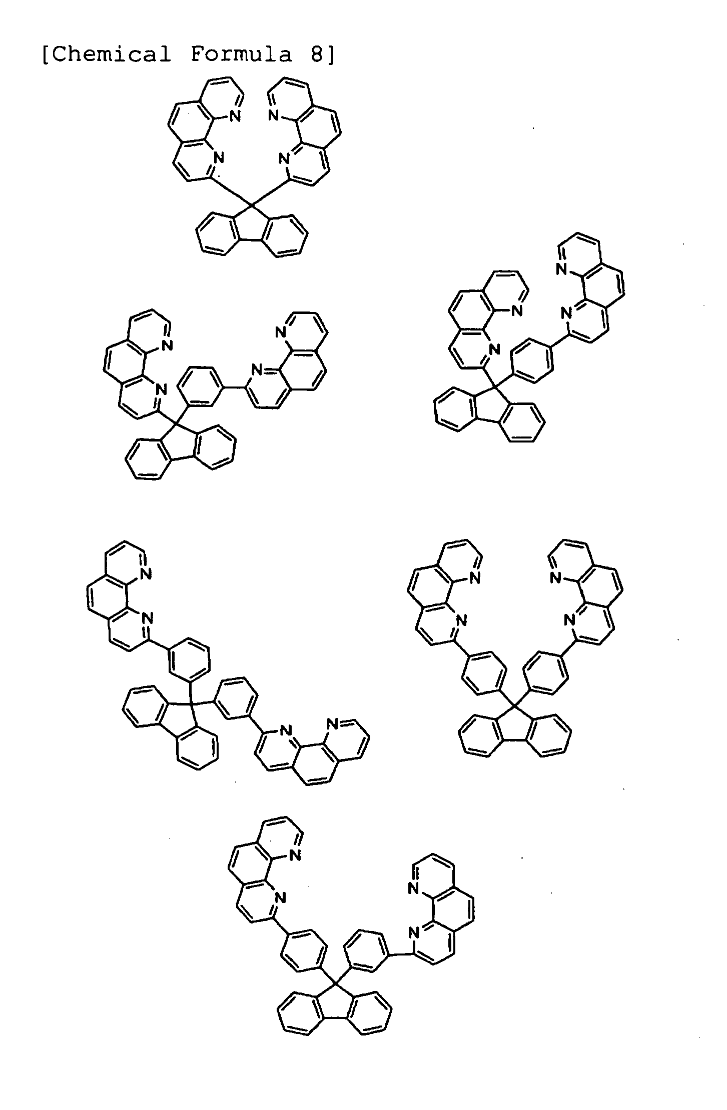

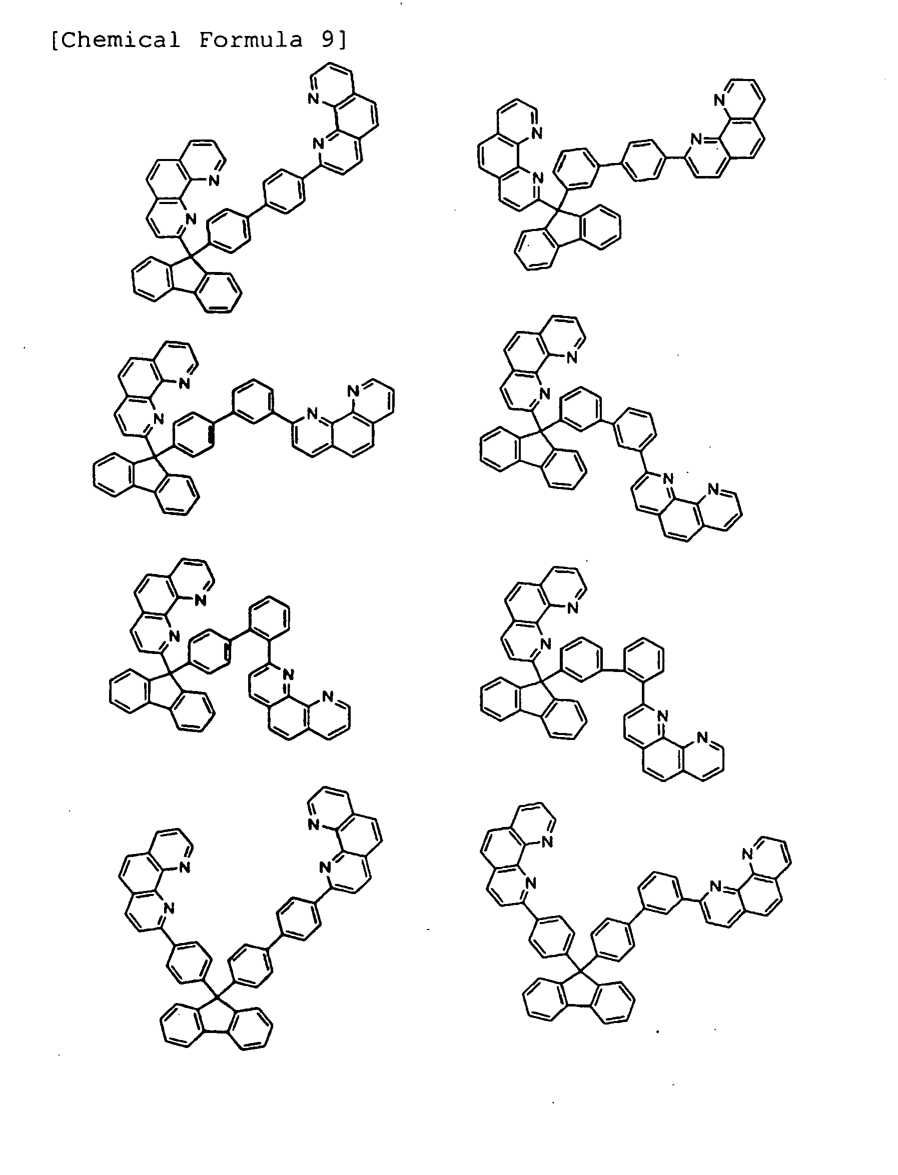

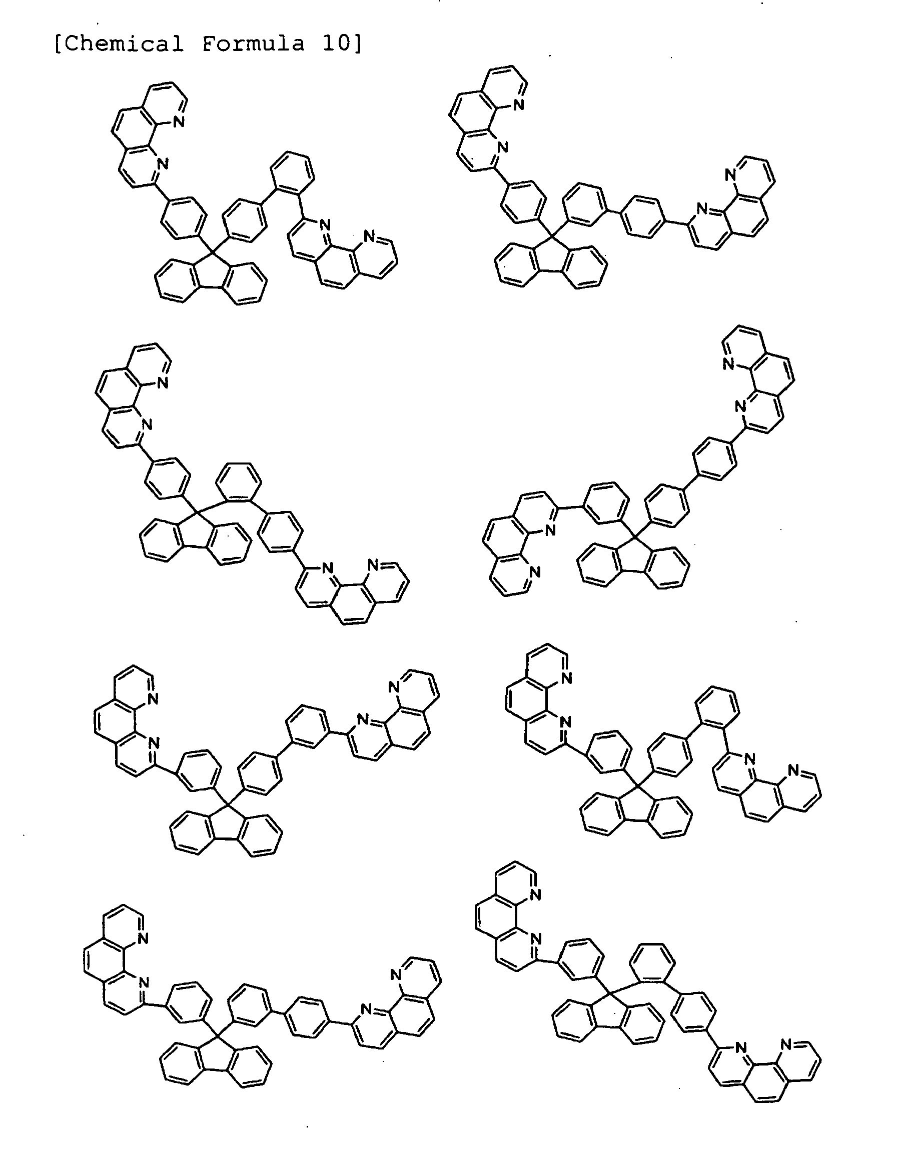

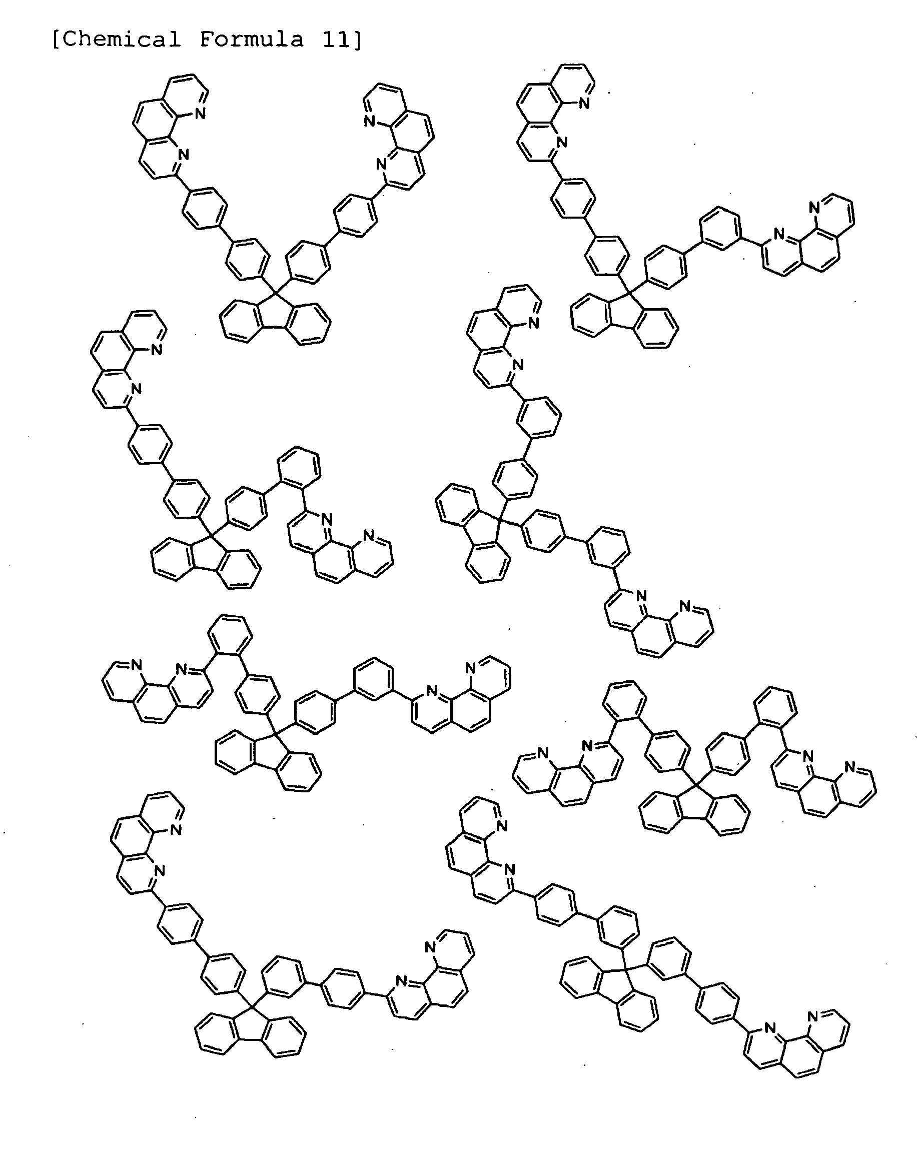

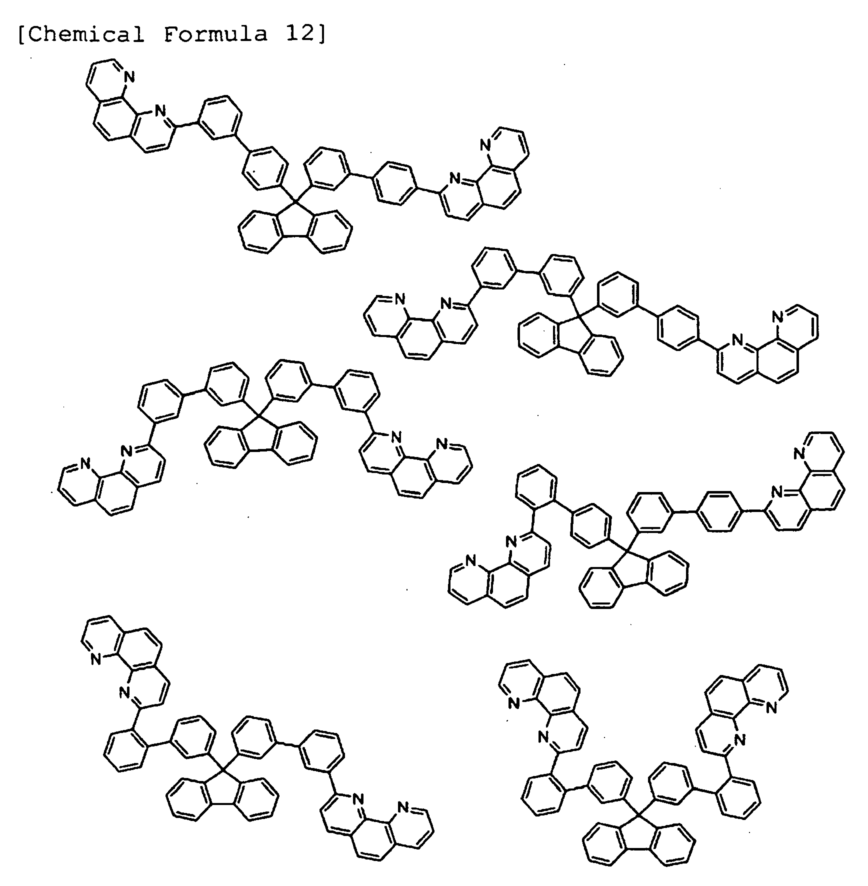

Concerning one typical structure among the above basic skeletons, examples of the structural formulae of the compounds expressed by the formulae under the title of Chemical Formula 7 and having any of the substituents R₁ to R₁₆ are illustrated below under the title of Chemical Formulae 13 to 18.

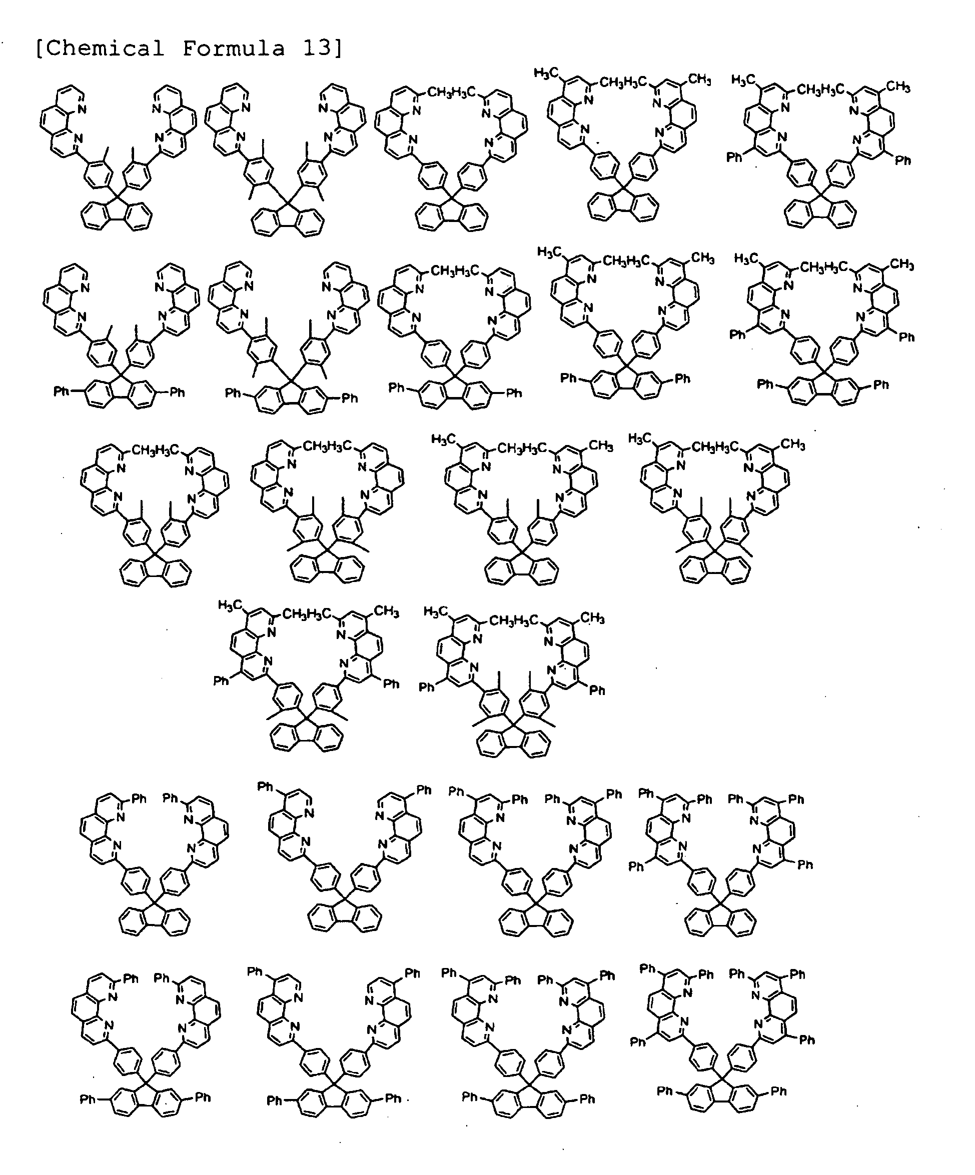

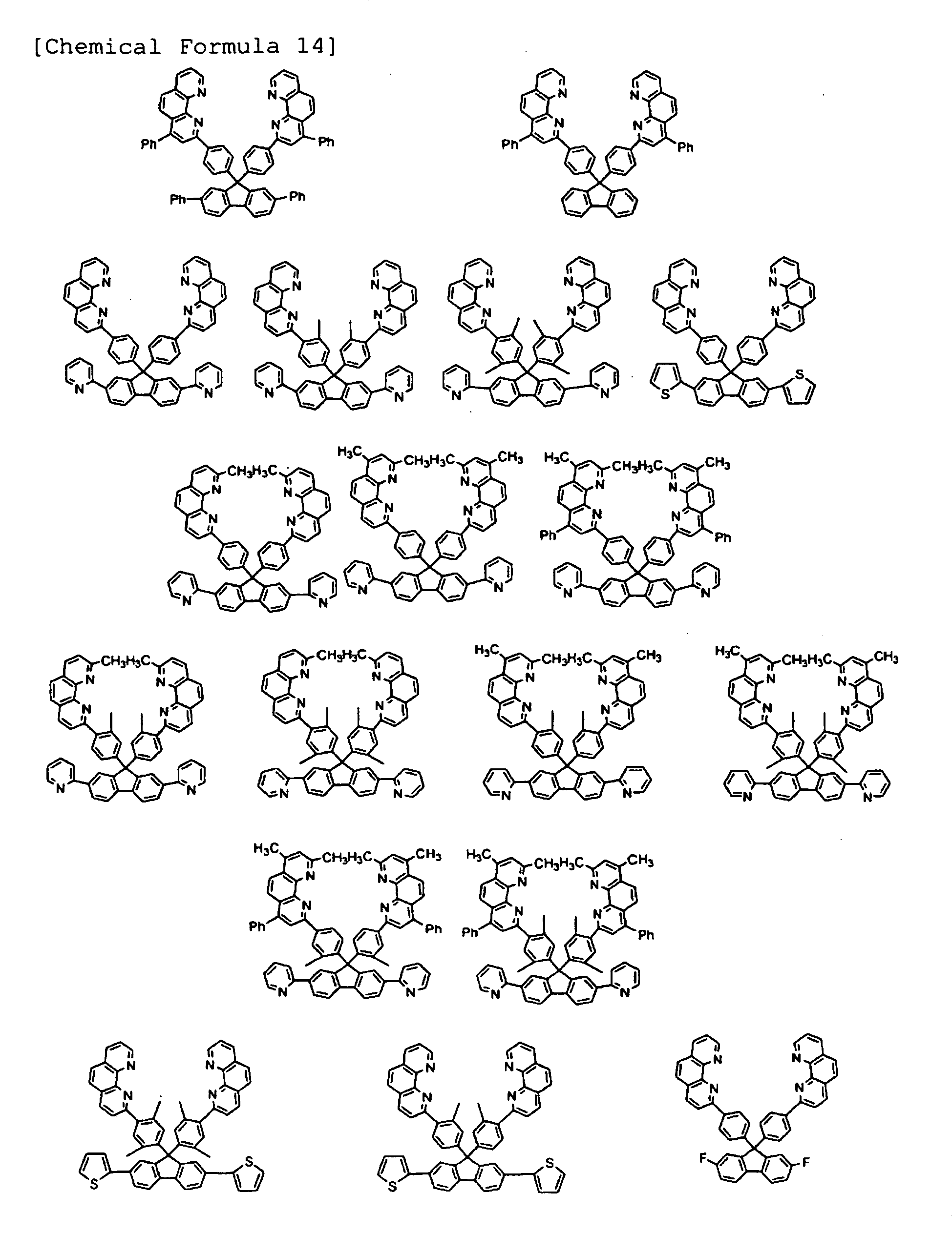

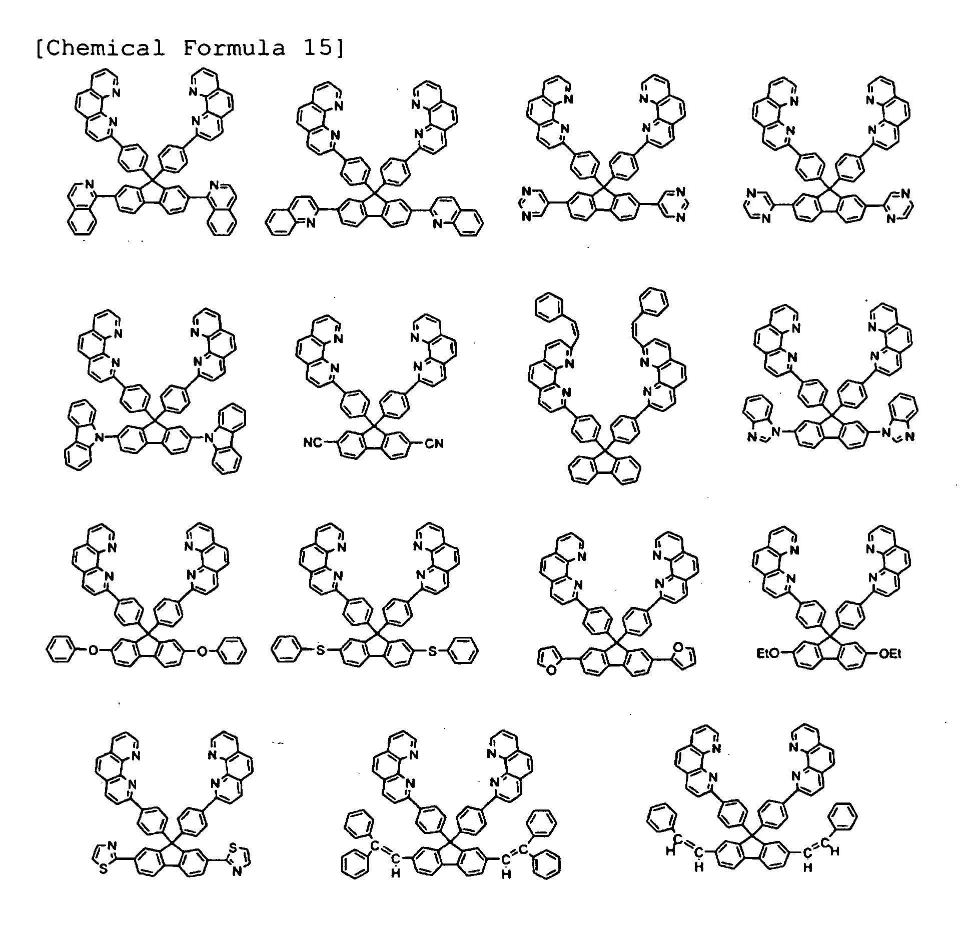

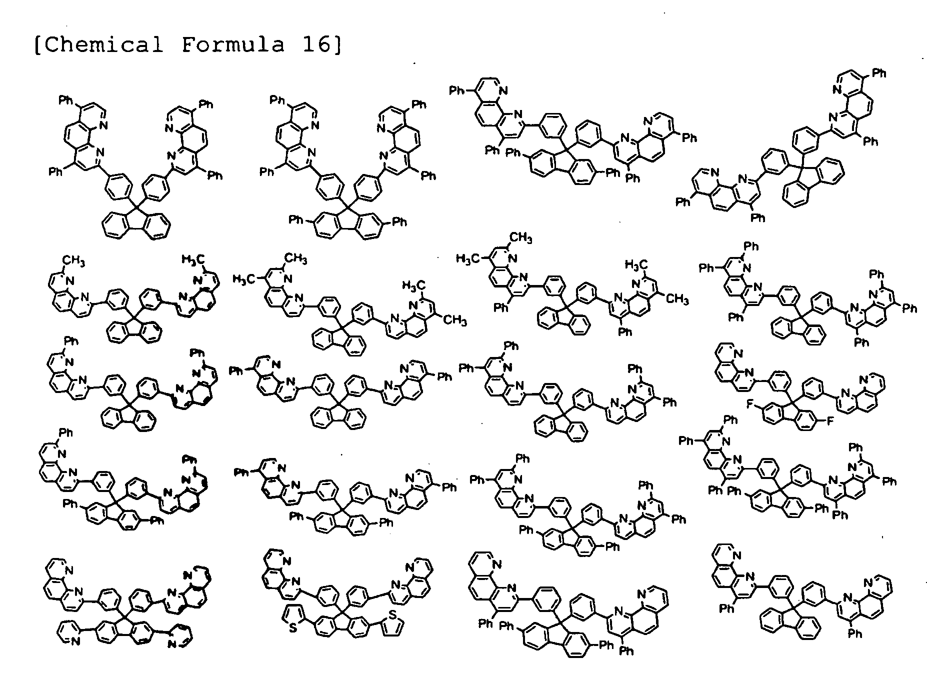

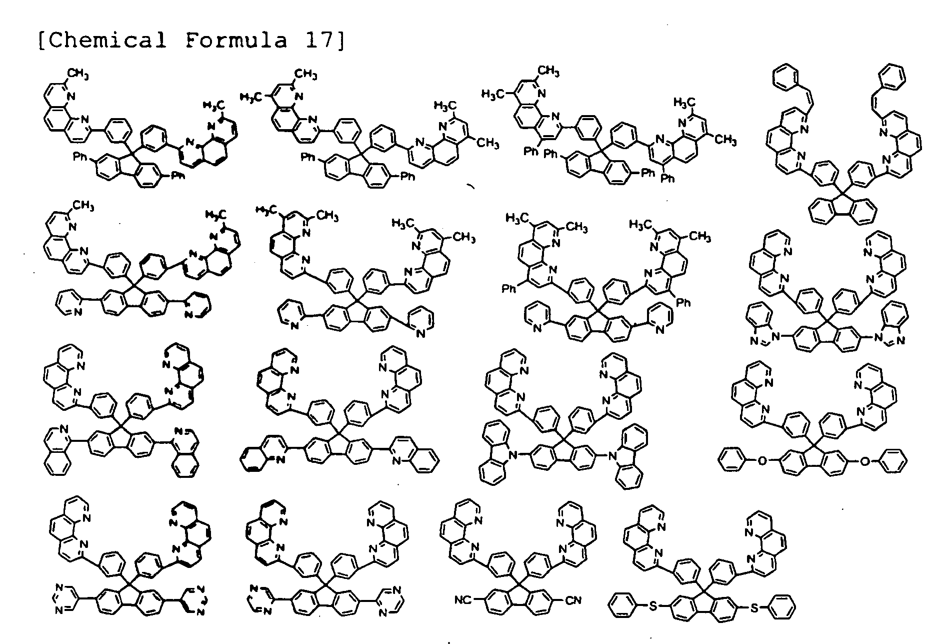

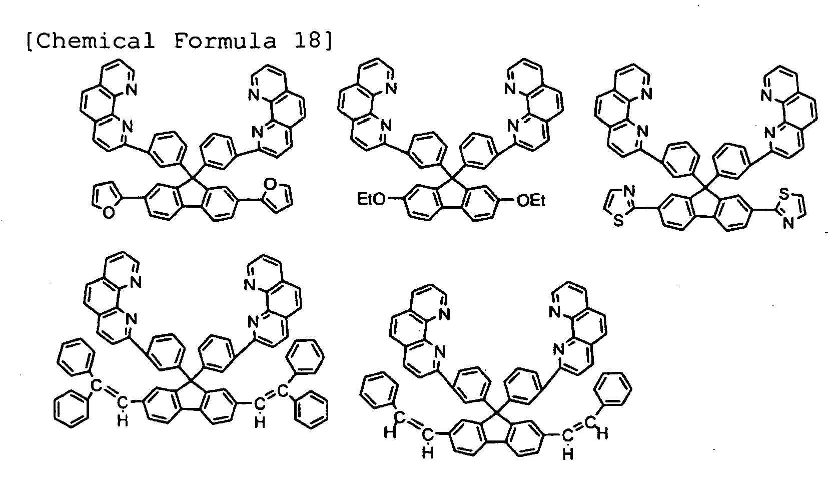

Among the compounds illustrated above under the titles of Chemical Formulae 13 to 18, DPF represented by Chemical Formula 5 or m-DPF represented by Chemical Formula 6 is particularly preferred.

In general, organic light-emitting devices are manufactured by a process that includes forming an organic thin film with an organic compound vapor from a heated vapor source. Therefore, organic compounds for use in organic light-emitting devices are required to have heat resistance such that they can withstand heating when used as vapor sources.

DPF and m-DPF each have a 5% weight loss temperature of 500°C or higher. These compounds have good thermal characteristics such that they can withstand heating when used as vapor sources.

As used herein, the term "weight loss temperature" indicates an index of the temperature at which organic compounds start to decompose. The higher the temperature, the higher the decomposition temperature, so that the heat resistance can be higher.

When organic light-emitting devices are designed, use in high temperature environments such as the interior of a car is taken into account. DPF and m-DPF each have a glass transition temperature (hereinafter abbreviated as Tg) and are amorphous. Therefore, device destruction due to crystallization of thin films can be suppressed using them. They can also withstand operation in high temperature environments, because they each have a high Tg of 170°C or higher.

As used herein, Tg indicates the scale of the phase transition temperature of organic compounds and can be observed for amorphous organic compounds. Amorphous organic compounds can form organic compound thin films of high quality. In particular, high-Tg organic compounds can form organic light-emitting devices in which thin films are highly maintainable in high temperature environments and less likely to be broken.

In contrast, bathophenanthroline and bathocuproin, which are conventionally used, have melting points of 218°C and 279°C (according to a catalog available from Aldrich), respectively, which are lower than that of DPF.

Phenanthroline derivatives represented by the formulae below under the title of Chemical Formula 19, such as DPB, all have a 5% weight loss temperature of 490°C or lower, which is lower than that of DPF, and provide no Tg but have crystallinity.

Therefore, the phenanthroline derivative used as the electron transporting material according to the invention has thermal characteristics higher than those of conventional phenanthroline derivatives.

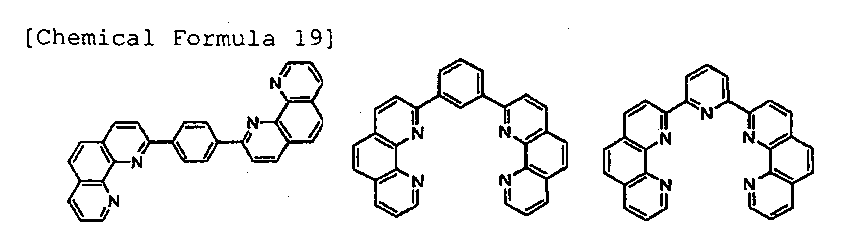

Specifically, DPF as a typical example of the electron transporting material of the invention may be synthesized by the method described in the section of Examples below. The starting material to be used, 8-amino-7-quinolinecarboaldehyde, may be synthesized according to the method described in JP-A No. 2004-311184.

The reaction solvent to be used for the synthesis of the electron transporting material of the invention is preferably an alcohol such as ethanol or isopropyl alcohol, an ether such as alkylene glycol monoalkyl ether or alkylene glycol dialkyl ether, or a polar aprotic solvent such as dimethylformamide (DMF) or DMSO. Above all, alkylene glycol monoalkyl ether such as ethylene glycol monoethyl ether (ethylcellosolve) is particularly preferred.

A reaction aid for alkalization may also be used. Examples of inorganic reaction aids include alkali metal salts such as sodium hydroxide and potassium hydroxide, alkaline earth metal salts such as barium hydroxide, hydroxide salts of alkali metals such as sodium and potassium, and hydroxide salts of alkaline earth metals such as barium. Examples of organic reaction aids include alcoholates of alkali metals such as sodium and potassium, alcoholates of alkaline earth metals such as calcium, and amine compounds such as triethylamine.

When nitrile compounds are synthesized, a polar aprotic solvent such as DMF, DMSO and NMP is preferably used as the reaction solvent, and DMF is particularly preferred. In this case, a metal carbonate such as sodium carbonate, potassium carbonate or cesium carbonate is preferably used as the reaction aid, and potassium carbonate or cesium carbonate is particularly preferred.

When acetyl compounds are synthesized, an ether solvent such as diethyl ether, THF or CPME is preferably used as the reaction solvent, and CPME or THF is particularly preferred.

The reaction for the synthesis of the electron transporting material of the invention may proceed in the air but is preferably performed under an inert gas such as nitrogen, helium, neon, or argon.

Organic light-emitting devices having the highly heat-resistant electron transporting material-containing layer described above can have thermal characteristics higher than those of conventional devices.

A single-layer type organic light-emitting device including the electron transporting material-containing layer may have a structure of first electrode, light-emitting layer and second electrode, in which the light-emitting layer may contain a hole-transporting luminescent material and the electron transporting material.

A two-layer type organic light-emitting device may include a first electrode, a light-emitting layer, a hole blocking layer, and a second electrode, in which the hole blocking layer may contain the electron transporting material. A multilayer type organic light-emitting device may include a first electrode, a hole transporting layer, a light-emitting layer, a hole blocking layer, and second electrode, in which the hole blocking layer may contain the electron transporting material.

The organic light-emitting device may also include an electron transporting layer that is placed between the hole blocking layer and the second electrode and adjacent to the hole blocking layer.

Specifically, the multilayer type organic light-emitting device may include a first electrode, a light-emitting layer, a hole blocking layer, an electron transporting layer, and a second electrode or a first electrode, a hole transporting layer, a light-emitting layer, a hole blocking layer, an electron transporting layer, and a second electrode.

The organic light-emitting device may also include a hole injecting layer that is placed between the first electrode and the hole transporting layer and adjacent to the hole transporting layer.

Specifically, the multilayer type organic light-emitting device may include a first electrode, a hole injecting layer, a hole transporting layer, a light-emitting layer, a hole blocking layer, and a second electrode or a first electrode, a hole injecting layer, a hole transporting layer, a light-emitting layer, a hole blocking layer, an electron transporting layer, and a second electrode.

A substrate as a component of the organic light-emitting device of the invention serves as a support for the organic light-emitting device. When the substrate side is the light-emitting side, a transparent substrate that is optically transparent to visible light should be used. The light transmittance thereof is preferably 80% or more, more preferably 85% or more, even more preferably 90% or more.

The transparent substrate is generally a glass substrate such as a substrate of optical glass such as BK7, BaK1 or F2, quartz glass, non-alkali glass, borosilicate glass, or aluminosilicate glass or a polymer substrate such as a substrate of acrylic resin such as PMMA, polycarbonate, polyethersulfone, polystyrene, polyolefin, epoxy resin, or polyester such as polyethylene terephthalate.

The thickness of the substrate is generally from about 0.1 to about 10 mm, preferably from 0.3 to 5 mm, more preferably from 0.5 to 2 mm, in view of mechanical strength, weight or the like.

In an embodiment of the invention, a first electrode is preferably formed on the substrate. The first electrode is generally an anode and generally made of a high work-function (4 eV or more) metal, alloy or electrically-conductive compound. The first electrode is preferably formed as a transparent electrode on the transparent substrate.

The transparent electrode is generally formed using a metal oxide such as indium tin oxide (ITO), indium zinc oxide or zinc oxide. In particular, ITO is preferably used in view of transparency, conductivity and so on.

The thickness of the transparent electrode is preferably from 80 to 400 nm, more preferably from 100 to 200 nm, in view of reliable transparency and conductivity.

The anode is generally formed by a sputtering method, a vacuum deposition method or the like and preferably formed as a transparent electrically-conductive thin film.

When the anode is the first electrode, the counter second electrode is a cathode that may be made of a low work-function (4 eV or less) metal, alloy or electrically-conductive compound, such as aluminum, an aluminum-lithium alloy or a magnesium-silver alloy.

The thickness of the cathode is preferably from 10 to 500 nm, more preferably from 50 to 200 nm.

The anode and the cathode may be each formed by a conventional film forming method such as a sputtering method, an ion plating method or a vapor deposition method.

The light-emitting layer of the organic light-emitting device of the invention is preferably made of a film comprising a molecular dispersion of components including at least a hole-transporting luminescent material and the electron transporting material of the invention.

The hole-transporting luminescent material may be a known material, examples of which include aromatic amine compounds such as triphenylamine derivatives, carbazole derivatives and styrylamine derivatives, stilbene derivatives, and distyryl derivatives.

In addition, the light-emitting layer including the hole-transporting luminescent material and the electron transporting material including the phenanthroline derivative according to the invention such as DPF may be doped with another luminescent material.

The luminescent material as a dopant may also be a known material, examples of which include condensed aromatic hydrocarbons such as anthracene, perylene, pyrene, and rubrene, coumarin derivatives, naphthalimide derivatives, perinone derivatives, complexes of rare earth metals such as Eu and Tb, dicyanomethylenepyran derivatives, dicyanomethylenethiopyran derivatives, acridine derivatives, acridone derivatives, rhodamine derivatives, cyanine dye derivatives, fluorescein derivatives, acridine derivatives, acridone derivatives, quinacridone derivatives, squarylium derivatives, iridium complexes, and platinum complexes.

The hole blocking layer may be formed using any known hole blocking material. The electron transporting material of the invention may also be used to form the hole blocking layer. The hole blocking layer may also be doped with a metal or a metal complex.

Examples of such a dopant include alkali metals such as Li, Na, K, and Cs, alkaline earth metals such as Ca and Ba, transition metals such as tungsten and rhenium, alkali metal complexes such as 8-quinolinol lithium (Liq), acetylacetone lithium (Liacac), dipivaloyl methane lithium (LiDPM), 8-quinolinol sodium (Naq), 8-quinolinol cesium (Csq), metal halides such as LiF and MgF, and metal oxides such as LiO₂, MgO and Al₂O₃.

Any known low-molecular or polymer materials is not particular limited but may be used to form the hole injecting layer, the hole transporting layer and the electron transporting layer.

Each of the above layers may be formed by various conventional film forming methods such as spin coating methods and vacuum deposition methods.

The thickness of each of the above layers may be appropriately determined depending on the conditions in view of compatibility between the layers, the desired total thickness and so on. In general, however, the thickness of each of the above layers is preferably in the range of 5 nm to 5 µm.

The invention is more specifically described with reference to the examples below, which are not intended to limit the scope of the invention.

### [Example 1]

### (Synthesis of DPF)

DPF was synthesized according to the synthesis scheme shown below under the title of Chemical Formula 20.

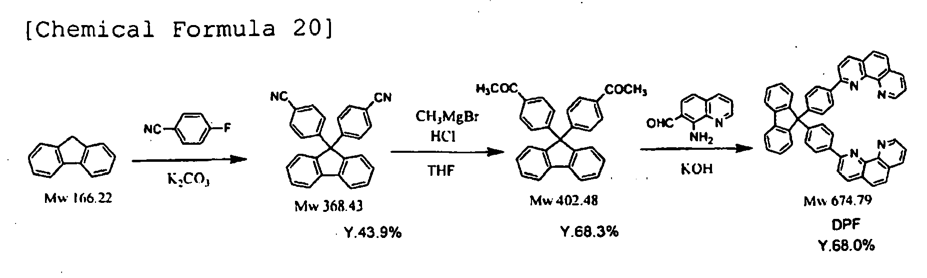

First, 12.5 g (75.39 mmol) of fluorene, 21.0 g (173.4 mmol) of 4-cyanofluorobenzene, DMF, and 1.0 g (7.52 mmol) of potassium carbonate were added in this order to a flask and allowed to react under a nitrogen atmosphere at 125°C for 22 hours.

The reaction solution was filtrated, and the resulting filtrate was subjected to reprecipitation with methanol. The precipitated crystal was separated by filtration, washed with methanol and dried in a dryer.

As a result of mass spectrometry (MS) and ¹H-NMR analysis, the resulting crystal was identified as the target product, 9,9'-bis(4-cyanophenyl)fluorene. The amount of the product was 12.2 g, and the yield was 43.9% based on the amount of fluorene.

Thereafter, 5.5 g (14.93 mmol) of the resulting 9,9'-bis(4-cyanophenyl)fluorene and TFT were added to a reaction vessel and stirred under a nitrogen atmosphere at 20°C to form a solution. To the solution was added dropwise 72 ml (71.66 mmol) of a 1 mol/l THF solution of methyl magnesium bromide over 10 minutes. The temperature was then raised, and the mixture was allowed to react at 50°C for 20 hours. After the reaction, the liquid temperature was lowered to 20°C, and dilute hydrochloric acid was added to the reaction solution.

The reaction solution was extracted with chloroform. After the chloroform layer was washed twice with water, the chloroform was removed so that a crude product was obtained.

The crude product was purified on a silica gel column developed with a mixed solvent of ethyl acetate/n-hexane.

As a result of MS and ¹H-NMR analysis, the purified product was identified as the target product, 9,9'-bis(4-acetylphenyl)fluorene. The amount of the product was 4.1 g, and the yield was 68.3% based on the amount of 9,9'-bis(4-cyanophenyl)fluorene. To a reaction vessel were added 3.0 g (7.453 mmol) of the resulting 9,9'-bis(4-acetylphenyl)fluorene, 2.6 g (15.28 mmol) of 8-amino-7-quinolinecarboaldehyde and a saturated ethanol solution containing ethylcellosolve and 1.3 g (23.85 mmol) of potassium hydroxide and allowed to react under a nitrogen atmosphere at 80°C for 23 hours.

The reaction solution was cooled to 20°C and poured into water and then extracted with chloroform. After the chloroform layer was washed twice with water, the chloroform was removed so that a crude product was obtained.

The crude product was purified on a silica gel column developed with a mixed solvent of chloroform/methanol and then further purified with a sublimation purification apparatus.

As a result of MS, ¹H-NMR and elemental analysis, the purified product was identified as the target product, DPF. The result of the elemental analysis was as follows: 86.94% C, 4.20% H and 8.30% N (Calc.: 87.22% C, 4.48% H and 8.30% N).

The amount of the product was 3.4 g, and the yield was 68.0% based on the amount of 9,9'-bis(4-acetylphenyl)fluorene.

The DPF synthesized as described above was subjected to thermal analysis.

The 5% thermal weight loss temperature measured by TG-DTA was 516.2°C.

The melting point and TG measured by DSC were 346.71°C and 203.85°C, respectively. In DSC scanning for the second or later time, Tg was observed, but no peak derived from the melting point was observed.

This suggests that the DPF should be amorphous.

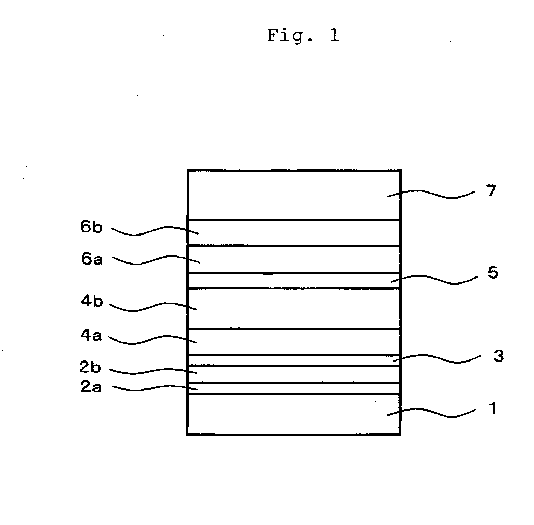

The DPF synthesized described above was used as an electron transporting material, and an organic light-emitting device having the layered structure shown in Fig. 1 was prepared by the method described below.

### (First Electrode)

A glass substrate with a 110 nm-thick patterned transparent electrically-conductive film (ITO) was subjected to ultrasonic cleansing with pure water and a surfactant, cleaning with running pure water, ultrasonic cleansing with a solution of a 1:1 mixture of pure water and isopropyl alcohol, and cleaning with boiling isopropyl alcohol, in this order. The substrate was slowly pulled out of the boiling isopropyl alcohol, dried in isopropyl alcohol vapor and finally subjected to ultraviolet-ozone cleaning.

The substrate was used as an anode 1 and placed in a vacuum chamber which was evacuated to 1 × 10⁻⁶ Torr. Molybdenum boats each charged with a material to be vapor-deposited and a vapor deposition mask for use in forming a film in a specific pattern were placed in the vacuum chamber. Each boat was heated by energization so that the material to be vapor-deposited was evaporated, when films were subsequently formed.

### (Hole Injecting Layer and Hole Transporting Layer)

NS21 (manufactured by Nippon Steel Chemical Co., Ltd.) was used as a hole transporting material. The respective boats were simultaneously heated by energization so that NS21 and molybdenum trioxide (MoO₃) were co-deposited by vapor deposition. As a result, a 10 nm-thick hole injecting layer 2a of 80:20 of NS21 : MoO₃ was formed.

A 20 nm-thick hole injecting layer 2b of 90:10 of NS21 : MoO₃ was then formed in the same manner, except that the composition ratio between NS21 and MoO₃ was changed.

A 5 nm-thick hole transporting layer 3 consisting of only NS21 was then formed.

### (Light-Emitting Layer)

In order to form a white light-emitting device, a 20 nm-thick light-emitting layer 4a of 98.7:1.3 of NS21:EY52 (manufactured by e-Ray Optoelectronics Technology Co., Ltd. (hereinafter abbreviated as "e-Ray")) was formed, and then a 30 nm-thick light-emitting layer 4b of 98.8:1.2 of EB43 (manufactured by e-Ray):EB52 (manufactured by e-Ray) was formed.

### (Hole Blocking Layer and Electron Transporting Layer)

A 5 nm-thick hole blocking layer 5 was formed of bis (2-methyl-8-quinolinolate) (p-phenylphenolate) aluminum (BAlq).

DPF was used as an electron transporting material to form a 14 nm-thick electron transporting layer 6a thereon. A 10 nm-thick electron transporting layer 6b was further formed of 74:26 of DPF:Liq thereon.

### Second Electrode

While the vacuum was maintained in the vacuum chamber, the mask was changed to a mask for vapor deposition of a cathode, and a 100 nm-thick aluminum (Al) layer was formed as a cathode 7.

The pressure in the vacuum chamber was returned to the atmospheric pressure, and the substrate with the layers each vapor-deposited as described above was taken out and transferred to a nitrogen-substituted glove box. The layers were sealed with UV-cured resin and another glass plate so that an organic light-emitting device was obtained.

Briefly, the layered structure of the device is as follows: ITO (110 nm) /NS21:MoO₃ (10 nm, 80:20) /NS21:MoO₃ (20 nm, 90:10) /NS21 (5 nm) /NS21:EY52 (20 nm, 98.7:1.3) /EB43:EB52 (30 nm, 98.8:1.2) /BAlq (5 nm) /DPF (14 nm) /DPF:Liq (10 nm, 74:26) /Al (100 nm).

### [Example 2]

### (Synthesis of DPF)

DPF was synthesized according to the synthesis scheme shown below under the title of Chemical Formula 21.

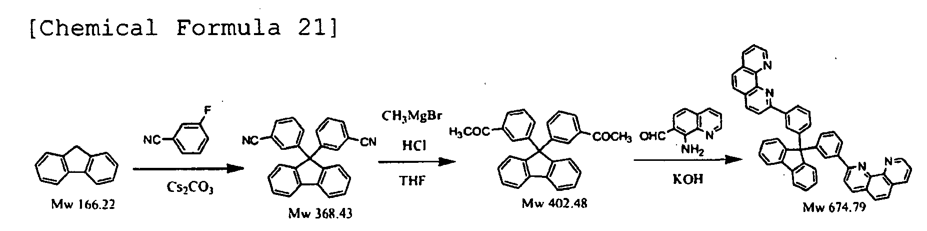

First, 5.0 g (30.08 mmol) of fluorene, 8.4 g (69.18 mmol) of 3-cyanofluorobenzene, 24.5 g (75.2 mmol) of cesium carbonate, and DMF were added in this order to a reaction vessel and allowed to react under a nitrogen atmosphere at 150°C for 22 hours.

The reaction solution was filtrated, and DMF was removed. The residue was then purified on a silica gel column developed with a mixed solvent of ethyl acetate/n-hexane.

As a result of MS and ¹H-NMR analysis, the purified product in the form of yellow sticky powder was identified as the target product, 9,9'-bis(3-cyanophenyl)fluorene. The amount of the product was 5.1 g (46.5% yield).

To a flask was added 70 ml of a 1 mol/l THF solution of methyl magnesium bromide, and the air was replaced by a nitrogen atmosphere. A solution of 5.1 g (14.01 mmol) of the resulting 9,9'-bis(3-cyanophenyl)fluorene in THF solution was injected thereto. The mixture was then allowed to react under the nitrogen atmosphere at the THF reflux temperature for 8.5 hours. After the reaction, the temperature was lowered to room temperature. Dilute hydrochloric acid was added dropwise to the reaction solution at room temperature, and a hydrolysis reaction was performed for 15 hours.

The reaction solution was extracted with chloroform. After the chloroform layer was washed twice with water, the chloroform was removed so that a crude product was obtained.

The crude product was purified on a silica gel column developed with a mixed solvent of ethyl acetate/n-hexane.

As a result of MS and ¹H-NMR analysis, the purified product was identified as the target product, 9,9'-bis(3-acetylphenyl)fluorene. The amount of the product was 1.9 g (33.9% yield).

To a reaction vessel were added 0.5 g (1.242 mmol) of the resulting 9,9'-bis(3-acetylphenyl)fluorene, 0.44 g (2.547 mmol) of 8-aminoquinoline-7-quinolinecarboaldehyde and ethylcellosolve in this order. To the resulting solution was added a solution of 0.22 g (3.974 mmol) of potassium hydroxide in ethanol solution, and the mixture was allowed to react under a nitrogen atmosphere at 75°C for 23.0 hours.

The reaction solution was filtrated, washed with ethanol, dried, and then purified on a silica gel column developed with a solvent of chloroform/methanol. The purified product was further purified with a sublimation purification apparatus.

As a result of MS, ¹H-NMR and elemental analysis, the purified product was identified as the target product, m-DPF. The result of the elemental analysis was as follows: 87.39% C, 4.41% H and 8.35% N (Calc.: 87.22% C, 4.48% H and 8.30% N).

The amount of the product was 0.6 g (71.6% yield).

The m-DPF synthesized as described above was subjected to thermal analysis.

Its 5% thermal weight loss temperature measured by TG-DTA was 500.0°C. Its melting point and Tg measured by DSC were 276.75°C and 172.53°C, respectively. In DSC scanning for the second or later time, Tg was observed, but no peak derived from the melting point was observed.

This suggests that the DPF should be amorphous.

### (reparation of Device)

An organic light-emitting device having the layered structure shown in Fig. 1 was prepared using the process of Example 1, except that the m-DPF synthesized as described above was used as the electron transporting material.

Briefly, the layered structure of the device is as follows: ITO (110 nm) /NS21:MoO₃ (10 nm, 80:20) /NS21:MoO₃ (20 nm, 90:10) /NS21 (5 nm) /NS21:EY52 (20 nm, 98.7:1.3) /EB43:EB52 (30 nm, 98.8:1.2) /BAlq (5 nm) /m-DPF (14 nm) /m-DPF:Liq (10 nm, 74:26) /Al (100 nm).

### [Example 3]

An organic light-emitting device having the layered structure shown in Fig. 1 was prepared using the process of Example 1, except that the m-DPF synthesized in Example 2 was used as the electron transporting material.

Briefly, the layered structure of the device is as follows: ITO (110 nm) /NS21:MoO₃ (10 nm, 80:20) /NS21:MoO₃ (20 nm, 90:10) /NS21 (5 nm) /NS21:EY52 (20 nm, 98.7:1.3) /EB43:EB52 (30 nm, 98.8:1.2) /m-DPF (20 nm) /m-DPF:Liq (10 nm, 74:26) /Al (100 nm).

### [Comparative Example 1]

Alq₃ was used as an electron transporting material, and an organic light-emitting device was prepared by the method described below.

### (First Electrode)

An anode was formed using the process of Example 1.

### (Hole Transporting Layer)

I200 (manufactured by Bando Chemical Industries, Ltd.) was used as a hole transporting material to form a 32.5 nm-thick hole transporting layer.

### (Light-Emitting Layer)

In order to form a white light-emitting device, a 20 nm-thick light-emitting layer of 98.9:1.1 of NS21:EY52 was formed, and then a 30 nm-thick light-emitting layer of 99.1:0.9 of EB43:EB52 was formed.

### (Electron Transporting Layer)

Alq₃ was used as an electron transporting material to form a 33.4 nm-thick electron transporting layer.

### (Second Electrode)

While vacuum was maintained in the vacuum chamber, the mask was changed to a mask for vapor deposition of a cathode, and a 0.5 nm-thick lithium fluoride (LiF) layer was formed, and then a 100 nm-thick Al layer was formed so that a cathode was obtained.

The pressure in the vacuum chamber was returned to the atmospheric pressure, and the substrate with the layers each vapor-deposited as described above was taken out and transferred to a nitrogen-substituted glove box. The layers were sealed with UV-cured resin and another glass plate so that an organic light-emitting device was obtained.

Briefly, the layered structure of the device is as follows: ITO (110 nm) /I200 (32.5 nm) /NS21+EY52 (20 nm, 98.9:1.1) /EB43+EB52 (30 nm, 99.1:0.9) /Alq₃ (33.4 nm) /LiF (0.5 nm) /Al (100 nm).

### (Luminous Efficiency Evaluation Test)

The luminous efficiency of each of the devices prepared in the examples and the comparative example was evaluated by angle-resolved emission intensity measurement of initial emission (current density: 100 A/m²).

The results are shown in Table 1.

**[Table 1]**

| | Electron Transporting Material | Voltage (V) | External Quantum Efficiency (%) | Luminous Efficiency (lm/W) | Energy Conversion Efficiency (%) |
|---|---|---|---|---|---|
| Example 1 | DPF | 3.7 | 5.3 | 11.0 | 3.3 |
| Example 2 | m-DPF | 4.1 | 5.3 | 10.6 | 2.9 |
| Example 3 | m-DPF | 4.0 | 5.2 | 10.8 | 3.0 |
| Comparative Example 1 | Alq₃ | 7.4 | 5.0 | 6.0 | 1.5 |

It is apparent from the evaluation results shown in Table 1 that the organic light-emitting device using DPF or m-DPF which has a 5% weight loss temperature of 500°C or higher and a glass transition temperature Tg higher than 170°C and thus has good heat resistance (Examples 1 to 3) shows a significantly reduced voltage and has a significantly improved luminous efficiency and high initial emission characteristics as compared with the device using a conventional electron transporting material, Alq₃ (Comparative Example 1).

It is also apparent from Example 3 that m-DPF has both hole blocking ability and electron transporting ability and thus is useful for a hole blocking layer accompanied by a metal complex-doped layer.

### (Life Evaluation Test at High Temperature)

The white light-emitting devices prepared in the examples and the white light-emitting device using DPB (the left side under the title of Chemical Formula 19) in the electron transporting layer were subjected to a life evaluation test in a thermostatic chamber at 76°C.

The results are shown in Table 2, in which "half life" is a time period that it takes to reduce the initial brightness by one-half.

**[Table 2]**

| | Electron Transporting Material | Initial Brightness (cd/m²) | Initial Voltage (V) | CIE Chromaticity Coordinate (x,y) | | Half Life (hr) |
|---|---|---|---|---|---|---|
| | | | | Initial Value | Half Value | |
| Example 1 | DPF | 1210 | 3.3 | (0.29, 0.36) | (0.26, 0.33) | 550 |
| Example 2 | m-DPF | 1300 | 3.2 | (0.35, 0.41) | (0.33, 0.39) | 600 |
| Example 3 | m-DPF | 1320 | 3.1 | (0.344, 0.401) | (0.330, 0.389) | 555 |
| Comparative Example 2 | DPB | 1250 | 3.1 | (0.33, 0.38) | (0.31, 0.37) | 400 |

The evaluation results shown in Table 2 indicate that the change in chromaticity is not significant and that the level of the change caused by heat should be relatively low. It is also apparent that the device using DPF or m-DPF (Examples 1 to 3) has a long half life and high thermal stability as compared with the device using DPB (Comparative Example 2).

### (observation of Vapor-Deposited Thin Film Surface)

A thin film of each of DPF, m-DPF and DPB which were used as electron transporting materials in the examples and Comparative Example 2 was formed by vapor deposition, and the surface thereof was observed with a differential interference microscope.

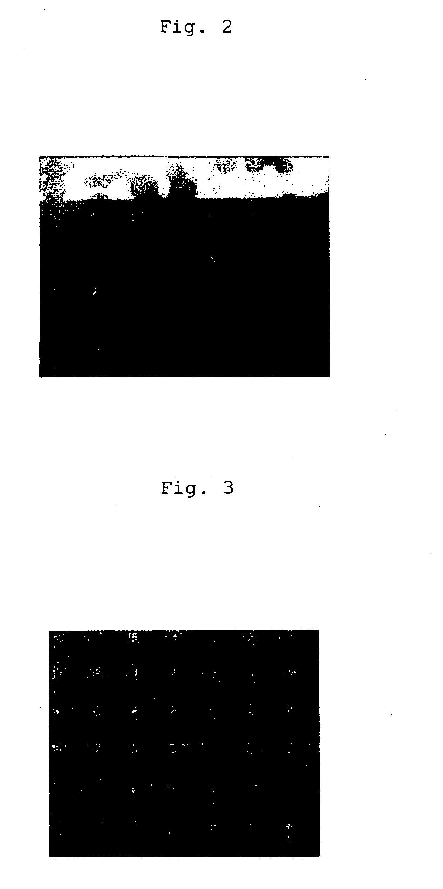

The respective micrographs (50×) are shown in Figs. 2 to 4. The actual color of the photographs of Figs. 2 to 4 is entirely blue. The part above the boundary line shows the vapor-deposited thin film on a glass, and the part below the boundary line shows the vapor-deposited thin film on ITO.

As is evident from the photograph of Fig. 2, spots potentially caused by crystallization were observed on the surface of the vapor-deposited DPB film immediately after the vapor deposition.

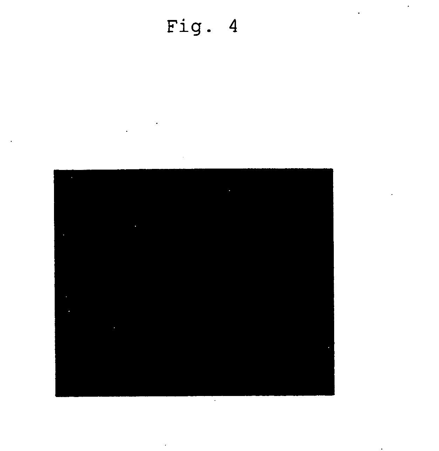

In contrast, as is evident from the photograph of Fig. 3 or 4, spots potentially caused by crystallization were not observed on the surface of the vapor-deposited film of each of DPF and m-DPF even 3 weeks after the vapor deposition.

This indicated that the DPF or m-DPF thin film was highly stable.

## Claims

1. Ah electron transporting material represented by Chemical Formula 1: 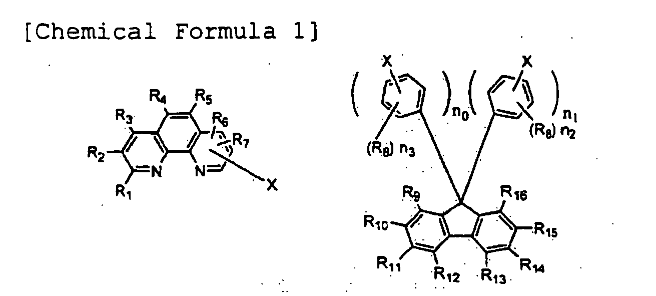 wherein R₁ to R₁₆ are each selected from hydrogen, alkyl, cycloalkyl, alkenyl, cycloalkenyl, alkynyl, aralkyl, aralkenyl, aralkynyl, aryl, cycloaryl, halogen, haloalkyl, haloalkenyl, haloalkynyl, haloaryl, heterocycle, alkoxy, aryloxy, hydroxyl, amino, alkylamino, arylamino, cyano, nitro, alkylthio, arylthio, aldehyde, carbonyl, carboxyl, ester, carbamoyl, silyl, and siloxanyl and may be the same or different; the position of the substituent R₆ or R₇ depends on the position of the linkage between the phenanthrolinyl group and the fluorenyl group; X represents a single bond between the phenanthrolinyl group and the fluorenyl group; n₀ and n₁ each independently represent an integer of 0 to 2; and n₂ and n₃ each represent the number of the substituents and each independently represent an integer of 1 to 4.

2. An electron transporting material according to Claim 1, which is represented by Chemical Formula 2:

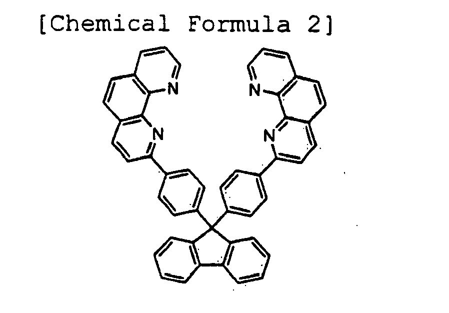

3. An electron transporting material according to Claim 1, which is represented by Chemical Formula 3:

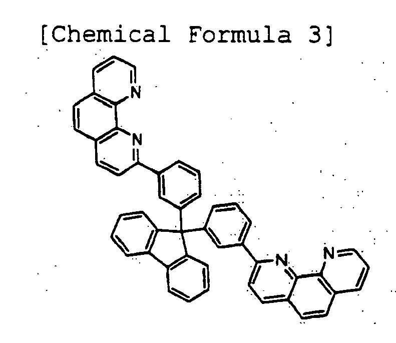

4. An organic light-emitting device, comprising a layer containing the electron transporting material according to any one of Claims 1 to 3.

5. The organic light-emitting device according to Claim 4, comprising a first electrode, a light-emitting layer and a second electrode placed in this order, the light-emitting layer comprising a hole-transporting luminescent material and the electron transporting material according to any one of Claims 1 to 3.

6. The organic light-emitting device according to Claim 4, comprising a first electrode, a light-emitting layer, a hole blocking layer, and a second electrode placed in this order, the hole blocking layer comprising the electron transporting material according to any one of Claims 1 to 3.

7. The organic light-emitting device according to Claim 4, comprising a first electrode, a hole transporting layer, a light-emitting layer, a hole blocking layer, and a second electrode placed in this order, the hole blocking layer comprising the electron transporting material according to any one of Claims 1 to 3.

8. The organic light-emitting device according to Claim 6 or 7, further comprising an electron transporting layer that is placed between the hole blocking layer and the second electrode and adjacent to the hole blocking layer.

9. The organic light-emitting device according to Claim 7, further comprising a hole injecting layer that is placed between the first electrode and the hole transporting layer and adjacent to the hole transporting layer.

10. The organic light-emitting device according to Claim 6 or 7, wherein the hole blocking layer is doped with a metal or a metal complex.

11. The organic light-emitting device according to Claim 8, wherein the electron transporting layer is doped with a metal or a metal complex.

12. The organic light-emitting device according to any one of Claims 5 to 7, wherein the light-emitting layer comprises a molecular dispersion of components comprising at least a hole-transporting luminescent material and the electron transporting material according to any one of Claims 1 to 3.

13. The organic light-emitting device according to Claim 12, wherein the light-emitting layer further comprises a luminescent material as a dopant.

14. The organic light-emitting device according to any one of Claims 5 to 7, wherein the first electrode comprises a transparent electrically-conductive thin film formed on a transparent substrate.

## Patentansprüche

1. Elektronentransportmaterial, dargestellt durch chemische Formel 1 : 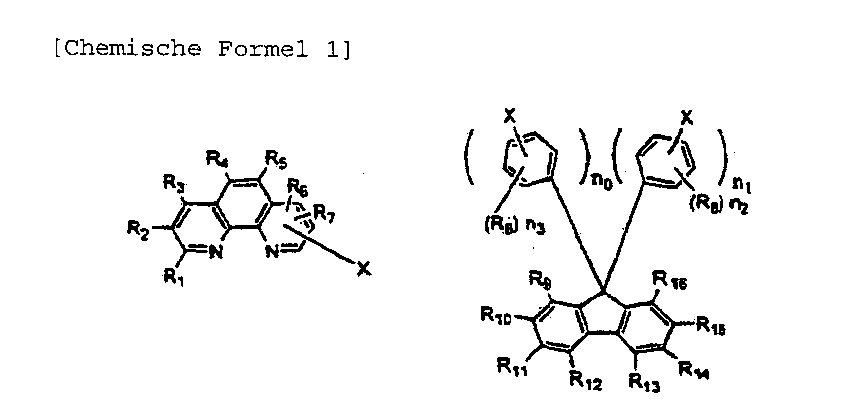 wobei R₁ bis R₁₆ jeweils ausgewählt sind aus Wasserstoff, Alkyl, Cycloalkyl, Alkenyl, Cycloalkenyl, Alkenyl, Aralkyl, Aralkenyl, Aralkinyl, Aryl, Cycloaryl, Halogen, Haloalkyl, Haloalkenyl, Haloalkinyl, Haloaryl, Heterocyclen, Alkoxy, Aryloxy, Hydroxyl, Amino,
Alkylamino, Arylamino, Cyano, Nitro, Alkylthio, Arylthio, Aldehyd, Carbonyl, Carboxyl, Ester, Carbamoyl, Silyl und Siloxanyl und können gleich oder verschieden voneinander sein; die Position des Substituenten R₆ oder R₇ ist abhängig von der Position von der Verknüpfungsgruppe zwischen der Phenanthrolinylgruppe und der Fluorenylgruppe; X ist eine Einfachbindung zwischen der Phenanthrolinylgruppe und der Fluorenylgruppe; n₀ und n₁ sind jeweils unabhängig voneinander ganze Zahlen von 0 bis 2; und n₂ und n₃ ist jeweils die Anzahl der Substituenten und unabhängig voneinander eine ganze Zahl von 1 bis 4.

2. Elektronentransportmaterial gemäß Anspruch 1, welches durch chemische Formel 2 dargestellt ist:

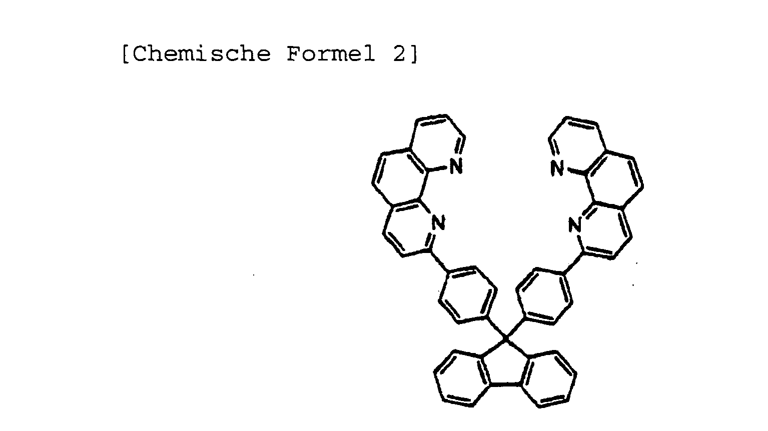

3. Elektronentransportmaterial gemäß Anspruch 1, welches durch chemische Formel 3 dargestellt ist:

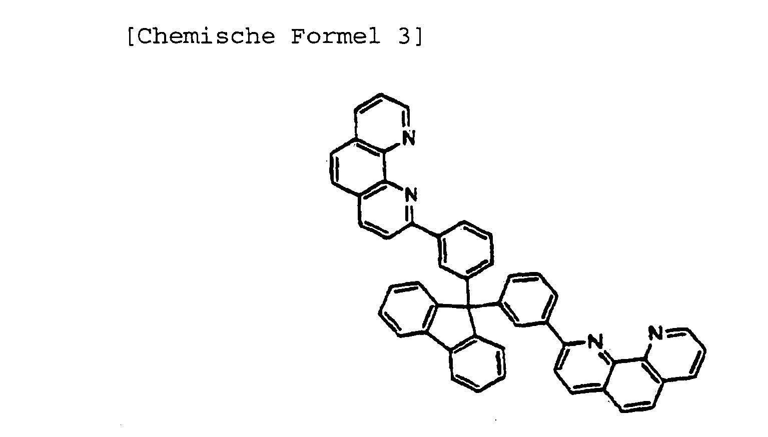

4. Organische lichtemittierende Vorrichtung, die eine Schicht umfasst, welche das Elektronentransportmaterial gemäß mindestens einem der Ansprüche 1 bis 3 enthält.

5. Organische lichtemittierende Vorrichtung gemäß Anspruch 4, die eine erste Elektrode, eine lichtemittierende Schicht und eine zweite Elektrode in dieser Reihenfolge umfasst, wobei die lichtemittierende Schicht ein Lochtransport-Lumineszenzmaterial und das Elektronentransportmaterial gemäß mindestens einem der Ansprüche 1 bis 3 umfasst.

6. Organische lichtemittierende Vorrichtung gemäß Anspruch 4, die eine erste Elektrode, eine lichtemittierende Schicht, eine lochblockierende Schicht und eine zweite Elektrode in dieser Reihenfolge umfasst, wobei die lochblockierende Schicht das Elektronentransportmaterial gemäß mindestens einem der Ansprüche 1 bis 3 umfasst.

7. Organische lichtemittierende Vorrichtung gemäß Anspruch 4, die eine erste Elektrode, eine lochtransportierende Schicht, eine lichtemittierende Schicht, eine lochblockierende Schicht und eine zweite Elektrode in dieser Reihenfolge umfasst, wobei die lochblockierende Schicht das Elektronentransportmaterial gemäß mindestens einem der Ansprüche 1 bis 3 umfasst.

8. Organische lichtemittierende Vorrichtung gemäß Anspruch 6 oder 7, die weiterhin eine Elektronen-transportierende Schicht umfasst, die zwischen der lochblockierenden Schicht und der zweiten Elektrode und angrenzend an die lochblockierende Schicht platziert ist.

9. Organische lichtemittierende Vorrichtung gemäß Anspruch 7, die weiterhin eine Lochinjektionsschicht umfasst, welche zwischen der ersten Elektrode und der lochtransportierenden Schicht und angrenzend an die lochtransportierende Schicht platziert ist.

10. Organische lichtemittierende Vorrichtung gemäß Anspruch 6 oder 7, wobei die lochblockierende Schicht mit einem Metall oder mit einem Metallkomplex dotiert ist.

11. Organische lichtemittierende Vorrichtung gemäß Anspruch 8, wobei die Elektronen-transportierende Schicht mit einem Metall oder mit einem Metallkomplex dotiert ist.

12. Organische lichtemittierende Vorrichtung gemäß mindestens einem der Ansprüche 5 bis 7, wobei die lichtemittierende Schicht eine Molekulardispersion umfasst, deren Komponenten mindestens ein lochtransportierendes Lumineszenzmaterial und das Elektronentransportmaterial gemäß mindestens einem der Ansprüche 1 bis 3 umfassen.

13. Organische lichtemittierende Vorrichtung gemäß Anspruch 12, wobei die lichtemittierende Schicht weiterhin ein Lumineszenzmaterial als Dotiermittel umfasst.

14. Organische lichtemittierende Vorrichtung gemäß mindestens einem der Ansprüche 5 bis 7, wobei die erste Elektrode einem transparenten elektrisch leitenden Dünnfilm umfasst, der auf einem transparenten Träger aufgetragen ist.

## Revendications

1. Matériau de transport d'électrons représenté par la formule chimique 1 : 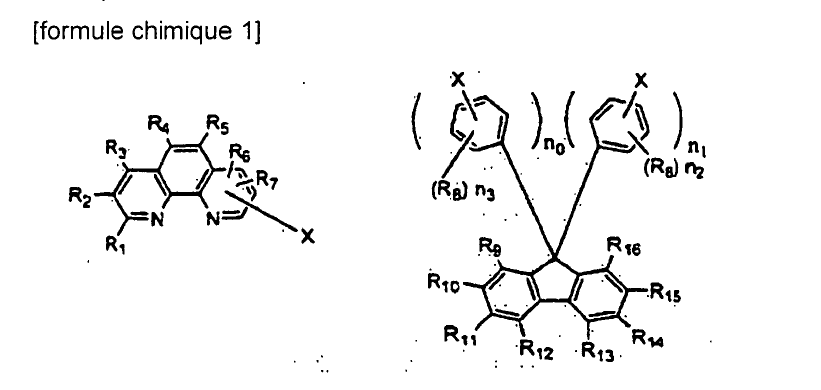 dans laquelle R₁ à R₁₆ sont sélectionnés chacun parmi l'hydrogène, un alkyle, un cycloalkyle, un alcényle, un cycloalcényle, un alcynyle, un aralkyle, un aralcényle, un aralcynyle, un aryle, un cycloaryle, un halogène, un halogénoalkyle, un halogénoalcényle, un halogénoalcynyle, un halogénoaryle, un hétérocyclique, un alcoxy, un aryloxy, un hydroxyle, un amino, un alkylamino, un arylamino, un cyano, un nitro, un alkylthio, un arylthio, un aldéhyde, un carbonyle, un carboxyle, un ester, un carbamoyle, un silyle, et un siloxanyle et peuvent être les mêmes ou différents ; la position du substituant R₆ ou R₇ dépend de la position de la liaison entre le groupe phénanthrolinyle et le groupe fluorényle ; X représente une liaison simple entre le groupe phénanthrolinyle et le groupe fluorényle ; n₀ et n₁ représentent chacun indépendamment un nombre entier de 0 à 2 ; et n₂ et n₃ représentent chacun le nombre de substituants et représentent chacun indépendamment un nombre entier de 1 à 4.

2. Matériau de transport d'électrons selon la revendication 1, qui est représenté par la formule chimique 2 :

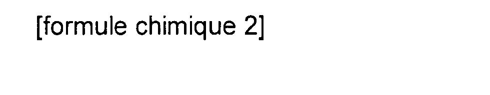

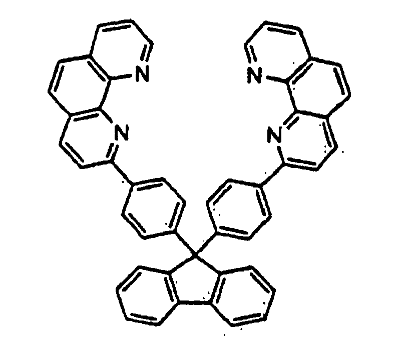

3. Matériau de transport d'électrons selon la revendication 1, qui est représenté par la formule chimique 3 :

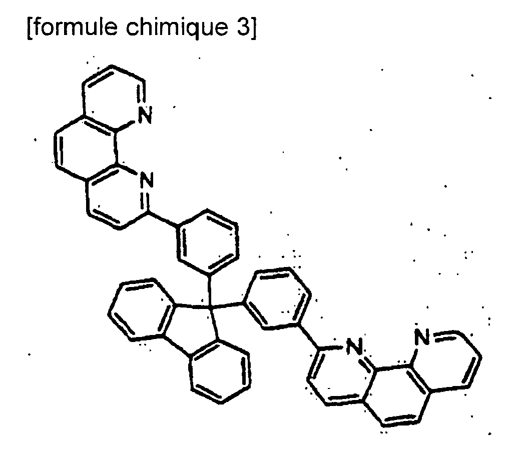

4. Dispositif électroluminescent organique, comprenant une couche contenant le matériau de transport d'électrons selon l'une quelconque des revendications 1 à 3.

5. Dispositif électroluminescent organique selon la revendication 4, comprenant une première électrode, une couche électroluminescente et une deuxième électrode placées dans cet ordre, la couche électroluminescente comprenant un matériau luminescent de transport de trous et le matériau de transport d'électrons selon l'une quelconque des revendications 1 à 3.

6. Dispositif électroluminescent organique selon la revendication 4, comprenant une première électrode, une couche électroluminescente, une couche de blocage de trous et une deuxième électrode placées dans cet ordre, la couche de blocage de trous comprenant le matériau de transport d'électrons selon l'une quelconque des revendications 1 à 3.

7. Dispositif électroluminescent organique selon la revendication 4, comprenant une première électrode, une couche de transport de trous, une couche électroluminescente, une couche de blocage de trous et une deuxième électrode placées dans cet ordre, la couche de blocage de trous comprenant le matériau de transport d'électrons selon l'une quelconque des revendications 1 à 3.

8. Dispositif électroluminescent organique selon la revendication 6 ou 7, comprenant en outre une couche de transport d'électrons qui est placée entre la couche de blocage de trous et la deuxième électrode et est adjacente à la couche de blocage de trous.

9. Dispositif électroluminescent organique selon la revendication 7, comprenant en outre une couche d'injection de trous qui est placée entre la première électrode et la couche de transport de trous et est adjacente à la couche de transport de trous.

10. Dispositif électroluminescent organique selon la revendication 6 ou 7, dans lequel la couche de blocage de trous est dopée avec un métal ou un complexe métallique.

11. Dispositif électroluminescent organique selon la revendication 8, dans lequel la couche de transport d'électrons est dopée avec un métal ou un complexe métallique.

12. Dispositif électroluminescent organique selon l'une quelconque des revendications 5 à 7, dans lequel la couche électroluminescente comprend une dispersion moléculaire de composants comprenant au moins un matériau luminescent de transport de trous et le matériau de transport d'électrons selon l'une quelconque des revendications 1 à 3.

13. Dispositif électroluminescent organique selon la revendication 12, dans lequel la couche électroluminescente comprend en outre un matériau luminescent en tant que dopant.

14. Dispositif électroluminescent organique selon l'une quelconque des revendications 5 à 7, dans lequel la première électrode comprend un film mince transparent électroconducteur formé sur un substrat transparent.
